# EUROPEAN PATENT SPECIFICATION

(11) **EP 0 713 373 B1**
(45) Date of publication and mention of the grant of the patent: **31.10.2001**
(21) Application number: 93918666.4
(22) Date of filing: 05.08.1993
(51) Int. Cl.: A61B 17/00, A61B 17/11

(54) **SIDE-TO-END VASCULAR ANASTOMOTIC STAPLE APPARATUS**
SEITE UND ENDE VERBINDENDE, VASKULARE ANASTOMOSEKLAMMERVORRICHTUNG
APPAREIL POUR APPLIQUER DES AGRAFES ANASTOMOTIQUES VASCULAIRES "COTE-VERS-EXTREMITE"

(43) Date of publication of application: 29.05.1996
(73) Proprietor: KASTER, Robert L., Plymouth, MN 55447 (US)
(72) Inventor: KASTER, Robert, L., Plymouth, MN 55447 (US); DOMAAS, Perry, M., Brooklyn Center, MN 55429 (US)
(74) Representative: Perani, Aurelio
(86) International application number: US9307377
(87) International publication number: WO9504503

(56) References cited:
- DE-A- 2 657 255
- US-A- 1 150 358
- US-A- 2 558 132
- US-A- 3 416 821
- US-A- 4 214 587
- US-A- 4 651 733
- US-A- 4 747 407

## Description

### BACKGROUND OF THE INVENTION

This invention relates generally to anastomotic connection of the ends of blood vessels to the sides of other blood vessels or other blood holding or transporting structures, such as the heart. The invention relates more particularaly to the use of stpales to realize such as anastomosis.

Anastomosis constitutes the joinder of parts or branches to allow intercommunication between them. In a surgical context, anastomosis occurs when a blood vessel becomes joined to another blood vessel or other blood filled object such that blood can flow freely through the established junction. Typically, side-to-end vascular anastomosis will be accomplished through use of sutures or specialized anastomotic connectors. These two options are often either time consuming or costly, and specialized connectors tend to expose nonorganic material in the area of blood flow.

Although simple mechanical fastening devices such as staples have been known for more than two centuries, and staples have been used in surgery since the 1920's, such use has been largely restricted to surgery of the gastrointestinal tract. Few attempts have been made to provide for anastomotic stapling of vascular members. The efforts that have been made to provide for a stapled vascular anastomosis appear to deal with end-to-end vascular anastomosis, and not side-to-end. The widespread use of staples for gastrointestinal tract surgery, as versus the limited or nonexistent use of staples for vascular surgery, can be easily explained. Blood vessels are typically comprised of remarkable thin tissues, whereas gastrointestinal tract tissues are generally fairly thick. Staples can be utilized with thicker materials in a relatively uncomplicated fashion. The use of staples for blood vessels, however, carries with it a high expected risk of rupture, tearing, or blood flow obstruction. As a result, even though stapling tools were provided in the early 1960's that would allow for end-to-end vascular anastomosis, this procedure is virtually unheard of in western medicine as of today. Side-to-end vascular anastomosis remains completely uncharted territory.

There exists a need for a side-to-end vascular anastomotic stapling apparatus that can be easily operated, will effectuate an anastomotic connection in a time and cost effective manner. The resulting stapled junction should minimize exposure of nonorganic material in the area of blood flow, and the anastomosis itself should be reliable and substantially free of risk of infection or other similar problems.

DE-A-2657255 discloses a surgical device for joining severed body vessel walls, which comprises a pair of annular flanges each formed of a plastic material which is compatible with the human body.

### SUMMARY OF THE INVENTION

These needs and others are substantially met through provision of the side-to-end vascular anastomotic stapling apparatus disclosed in this specification. The apparatus is comprised very generally of a stapling tool, a staple forming tool and staple. The apparatus has been particularly designed for use in side-to-end anastomosis, as where the end of a blood vessel becomes connected to the side or wall of a second blood vessel or other structure, such as the heart.

The stapling tool may be comprised in general of a sleeve, a core unit, a mandrel and a trigger unit.

The core unit may be formed of a plastic tube having a hole axially disposed through its entire length. The rearward end of the sleeve has a cavity formed peripherally thereabout for use in interfacing with the trigger unit. The forward end has an annularly shaped concave cavity formed therein to serve as an anvil.

The core unit may be comprised of a stainless steel tube that also has a hole axially disposed through its entire length. A plurality of slots are formed through its surface and parallel to its axis for a substantial portion of its length, thereby forming a plurality of fingers. Each finger has a groove formed therein and disposed substantially parallel to the slots and the axis of the core unit. So configured, the fingers are flexible in a radial direction. Finally, the core unit has a slot formed peripherally about its forward end to serve as a staple holding unit.

The exterior diameter of the core unit should be approximately the same as the interior diameter of the hole disposed axially through the sleeve, such that the core unit slidably disposed within the sleeve.

The mandrel may be comprised of a metal tube having a hole axially disposed through its entire length. The exterior diameter of the mandrel should be such that it may be slidably passed through the hole disposed axially through the core unit. To facilitate such sliding, a plastic tab may be affixed to one end of the mandrel to serve as a mandrel control unit.

The trigger unit includes a handle, a trigger and a bias unit. The handle affixes to the rearward end of the core unit. The trigger may be comprised of a movable plate that pivotally connects with respect to the sleeve and that interacts with the sleeve to cause the sleeve to move forward when the trigger is urged towards the handle. The bias unit includes a spring disposed between the handle and the trigger to urge the handle and trigger apart from one another.

The staple forming tool may be comprised of a plastic rod having two tapered ends. The first tapered end has a small hole formed therein. The second end has a small concave cavity formed therein.

The staples are each comprised of 316 surgical steel having a thickness of approximately .25 mm. (.010 inches). Each staple has a plurality of vessel and interior wall engaging members, and a plurality of exterior wall engaging members. In addition, in the embodiment disclosed in this specification, a connecting unit serves to operably join both groups of members.

In use, a staple may be mounted on the stapling tool by positioning the connecting unit of the staple in the staple holding unit on the core unit. The staple should be positioned with the plurality of vessel and interior wall engaging members oriented forwardly and with the plurality of exterior wall engaging members oriented rearward. With the mandrel introduced fully through the core unit, the forward end of the mandrel will extend slightly beyond the core unit and coincidentally cause the fingers of the core unit to be outwardly disposed such that the staple will be held firmly in place.

A blood vessel (either biologic or artificial, in humans or in animals) may be disposed through the hole provided in the mandrel. With the blood vessel extending somewhat beyond the forward end of the mandrel, the blood vessel tissue may be everted back over the staple such that the plurality of vessel and interior wall engaging members pierce the blood vessel and extend therethrough. The staple forming tool may then be utilized to bend the vessel and interior wall engaging members back upon themselves.

Following this, the apparatus may be positioned to dispose the staple and blood vessel combination at the already provided hole in the wall of the blood vessel or other structure to which the blood vessel is to be connected. The tip of the apparatus, including the staple, may be pushed through this hole, and then pulled backwards to cause the vessel and interior wall engaging members that are already piercing the blood vessel to also pierce and engage the interior wall of the second blood vessel or other structure.

While in this position, the trigger unit may be manipulated to cause the sleeve to move forward. This will cause the anvil positioned at the forward end of the sleeve to contact the exterior wall engaging members and urge them into a position to pierce and engage the exterior wall of the second blood vessel or other structure.

Following this, the mandrel may be removed by gripping the mandrel control unit and sliding it backwards through the core unit. Removal of the mandrel allows the fingers of the core unit to retract slightly, thus releasing their grip on the staple. The entire stapling tool may then be retracted from the area of the stapled anastomosis, and the anastomosis will be completed.

### BRIEF DESCRIPTION OF THE DRAWINGS

These and other attributes of the invention will become more clear upon a thorough review and study of the following description of the best mode for carrying out the invention, particularly when reviewed in conjunction with the drawings, wherein:
Fig. 1 comprises a perspective view of the stapling tool;
Fig. 2 comprises a side elevational view of the mandrel;
Fig. 3 comprises a side elevational view of the sleeve;
Fig. 4 comprises a front elevational view of the sleeve;
Fig. 5 comprises a side elevational view of the core unit;
Fig. 6 comprises a front elevational view of the core unit;
Fig. 7 comprises a side elevational, sectioned, detailed view of the core unit;
Fig. 8 comprises an enlarged front elevational view of a staple;
Fig. 9 comprises an enlarged side elevational view of a staple;
Fig. 10 comprises an enlarged front elevational view of a formed staple;
Fig. 11 comprises a side elevational view of the staple forming tool;
Fig. 12 comprises an enlarged detailed view of one end of the staple forming tool;
Fig. 13 comprises an enlarged detailed view of the staple forming tool;
Fig. 14 comprises an enlarged side elevational detail view of the staple on the stapling tool;
Fig. 15 comprises an enlarged side elevational detail view of the staple on the stapling tool with a blood vessel everted over it;
Fig. 16 comprises an enlarged side elevational detail view of the staple on the stapling tool with the vessel and interior wall engaging members bent rearwardly;
Fig. 17 comprises an enlarged side elevational detail view of the staple and blood vessel combination being disposed through a hole in a wall;
Fig. 18 comprises an enlarged side elevational detail view of the staple and blood vessel combination being retracted against the interior side of the wall; and
Fig. 19 is a view like Fig. 18, but showing the second set of exterior wall engaging members being pushed into their final position.

### DETAILED DESCRIPTION OF THE PREFERRED EMBODIMENTS

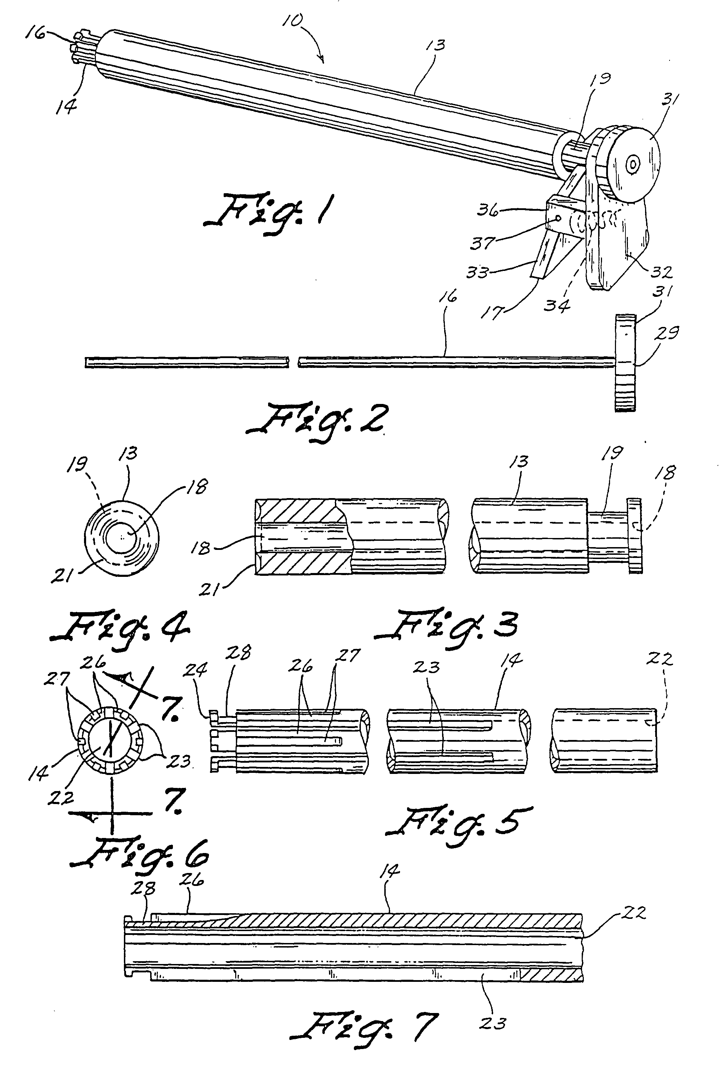

Referring now to the drawings, the apparatus can be seen to be generally comprised of a stapling tool (10) (Fig. 1), a staple forming tool (11) (Fig. 11), and a staple (12) (Fig. 10). Referring to Fig. 1, the stapling tool (10) includes generally a sleeve (13), a core unit (14), a mandrel (16) (Fig. 2), and a trigger unit (17). Each of these generally described components of the stapling tool (10) will now be described in more detail in seriatim fashion.

Referring to Figs. 3 and 4, the sleeve (13) may be comprised of a plastic tube having a hole (18) axially disposed therethrough. The rearward end of the sleeve (13) has a cavity (19) formed about the periphery thereof for interaction with the trigger unit as disclosed below. The forward end of the sleeve (13) has a concave shaped cavity (21) formed annularly about the hole (18). This cavity (21) serves as an anvil for the staple (12) described below.

In the embodiment depicted, the sleeve (13) has an overall length of approximately 141.91 mm. (5.587 inches) and a diameter of approximately 9.53 mm. (.375 inches). The hole (18) has a diameter of approximately 3.96 mm. (.156 inches).

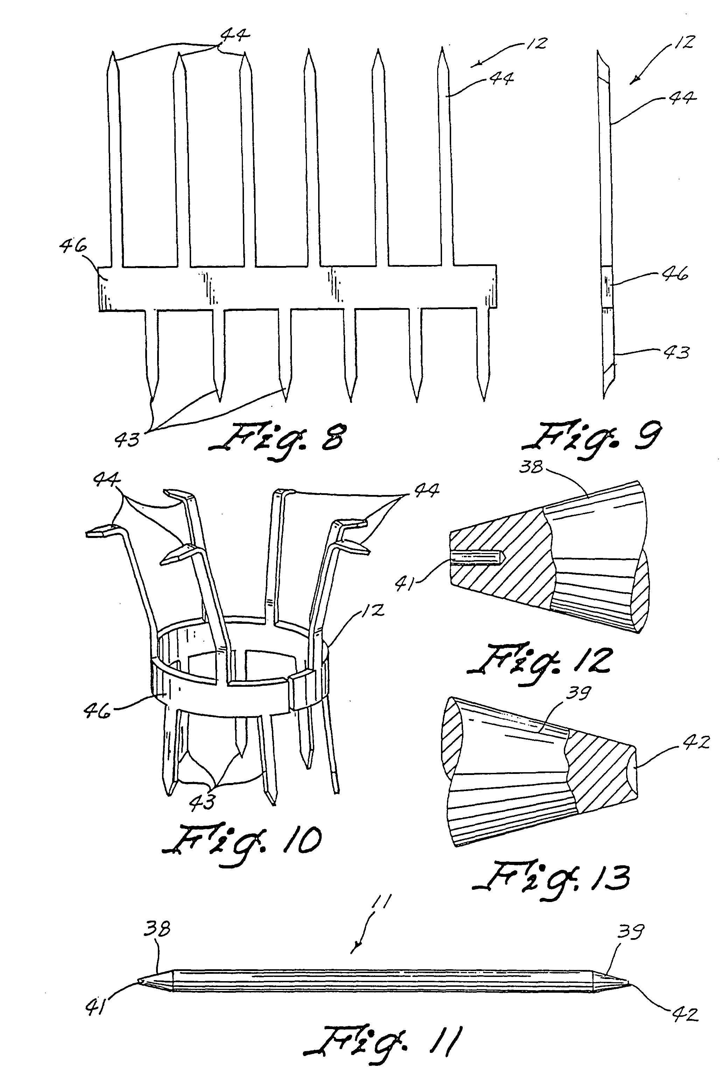

Referring now to Figs. 5, 6 and 7, the core unit (14) may be formed of a stainless steel tube having a hole (22) formed axially therethrough. A plurality of slots (23) are formed through the surface of the forward end of core unit (14) and are disposed substantially parallel to the axis of the core unit (14). These slots extend for an approximate length of 25 mm. from the forward end (24) of the core unit (14). By provision of these slots (23) a plurality of fingers (26) are formed. In the embodiment depicted, six slots (23) are provided to yield six fingers (26). Each finger (26) has a groove (27) formed therein and disposed substantially parallel to the slots (23) and the axis of the core unit (14). So configured, the fingers (26) are flexible in a radial direction.

Finally, it may be noted that a slot (28) has been formed about the periphery of the core unit (14) proximal the forward end (24) thereof. This slot (28) serves as a staple holding unit as disclosed in more detail below.

The core unit (14) has an overall length of approximately 152.40 mm. (6.0 inches) and a diameter of approximately 3.96 mm. (.156 inches). So dimensioned, the core unit (14) can be slidably disposed within the hole (18) provided through the sleeve (13). The hole disposed through the core unit (14) has a diameter of approximately 2.54 mm. (.10 inches)

Referring now to Fig. 2, the mandrel (16) may be comprised of a stainless steel tube having a hole (29) disposed axially therethrough. A mandrel control unit (31) comprising a plastic handle may be affixed to the rearward end thereof to allow the mandrel to be more easily grasped.

The mandrel tube has an overall length of approximately 157.15 mm. (6.187) inches and a diameter of approximately 2.54 mm. (.10 inches). So configured, the mandrel (16) can be slidably inserted through the hole (22) provided through the core unit (14). In addition, when fully inserted into the core unit (14), the mandrel (16) will extend slightly beyond the forward end (24) of the core unit (14). The inner hole (29) of the mandrel (16) may be of an appropriate dimension to accommodate the desired sized blood vessels that will be used with the stapling tool (10).

Referring again to Fig. 1, the trigger unit (17) includes generally a handle (32), a trigger (33) and a bias unit (34). The handle (32) may be comprised of a plastic tab that may be connected to the rearward end of the core unit (14). Two flanges (36) are attached to either side of the handle (32) and extend forwardly. A pivot rod (37) connected between each flange (36) and the trigger (33). The trigger (33) may be comprised of another plastic tab having a notch formed in it for allowing it to be positioned about the sleeve (13) and having holes disposed therethrough to accommodate the pivot rod (37). The bias unit (34) may be comprised of a normally expanded spring that fits between the trigger (33) and the handle (32).

So configured, it will be appreciated that the biasing unit (34) will normally urge the trigger (17) away from the handle (32). The trigger (33) may, however, be selectively urged rearwardly towards the handle (32). When this happens, the trigger (33) will pivot about pivot rod (37), and this will cause the upward edges of the trigger (33) to contact the notched portion of the sleeve (13). This in turn will cause the sleeve (13) to move forwardly with respect to the core unit (14). The significance of this motion will be made more clear below.

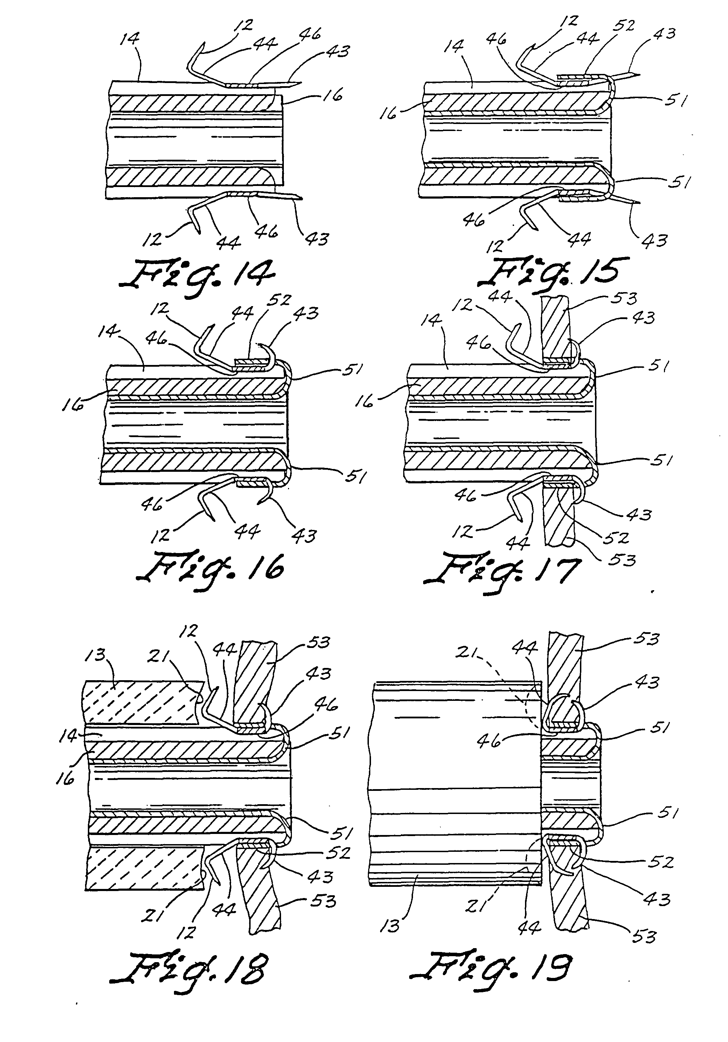

Referring now to Figs. 11, 12 and 13, the staple forming tool (11) will be described. The staple forming tool may be comprised of a plastic rod having two cone shaped ends (38) and (39). Referring to Fig. 12, the first cone shaped end (38) has a narrow tube shaped hole (41) formed therein. Referring to Fig. 13, the second cone shaped end (39) has a concave cavity (42) formed therein.

The staple forming tool (11) has an overall length of approximately 127.0 mm. (5.0 inches). The hole (41) formed in the first end (38) of the staple forming tool (11) has a depth of approximately 1.27 mm. (050 inches) and a diameter of approximately .396 mm. (.0156). The use of the staple forming tool (11) will be made more clear below.

Referring now to Figs. 8 and 9, the staple (12) may be formed of 316 stainless steel having a thickness of approximately .25 mm. (,010 inches). The staple includes a plurality of vessel and interior wall engaging members (43) and a plurality of exterior wall engaging members (44). Each of these members (43) and (44) are operably joined in this embodiment through use of a connecting unit (46) comprising a band. It will be appreciated that the vessel and interior wall engaging members (43) and the exterior wall engaging members (44) are all pointed and otherwise contoured to assure proper stapling action. The staple (12) may be formed through standard photo etching procession.

In this particular embodiment, the staple has an overall length of approximately 9.891 mm. (.3894 inches). The vessel and interior wall engaging members (43) are approximately 2.34 mm. (0.92 inches) in length, and the exterior wall engaging members (44) are approximately 5.28 mm. (.208 inches) in length.

Once formed in the manner described above, the staple (12) may be curved about a mandrel or other appropriate object to cause the connecting unit (46) to assume an annular shape as depicted in Fig. 10. Prior to the anastomotic procedure, the exterior wall engaging members (44) should each be bent twice through use of the staple forming tool (11) in the manner indicated, with the distal end curved at approximately 90° and the end more proximal to the connecting unit (46) bent slightly outward.

The method of using the apparatus may now be described.

A staple (12) (Fig. 14) may be positioned on the forward end (24) of the core unit (14) such that the connecting unit (46) of the staple (12) becomes lodged in the staple holding unit (28) of the core unit (14) with the vessel and interior wall engaging members (43) disposed forwardly and the exterior wall engaging members (44) disposed rearwardly. The mandrel (16) may then be fully disposed through the core unit (14). This positioning of the mandrel (16) will urge the fingers (26) of the core unit (14) slightly outward, and this will cause the staple (12) to be firmly held in place.

The end of the blood vessel to be connected to the wall of a second blood vessel or other structure may then be connected to a suture (not shown) in a manner well known to those skilled in the art of surgery. The suture may be easily inserted through the hole (29) in the mandrel (16) to cause the blood vessel to become similarly disposed therethrough. The blood vessel should be drawn through to extend approximately 3 mm. to 4 mm. beyond the forward end of the mandrel (16). The sutured end of the blood vessel may then be trimmed to remove the suture.

With reference to Fig. 15, the exposed end of the blood vessel (51) may then be everted back over the vessel and interior wall engaging members (43) such that the vessel and interior wall engaging members (43) pierce the blood vessel (51). The rearward end of the blood vessel may then be gently pulled in a rearward direction to firmly anchor the blood vessel (51) over the vessel and interior wall engaging members (43).

Following this, the hole (41) provided in the first end (38) of the staple forming tool (11) may be operably joined with each vessel and interior wall engaging member (43) such that an operator may cause the vessel and interior wall engaging members (43) to be bent radially approximately 90°. The second end (39) of the staple forming tool (11) may then be utilized to further urge the vessel and interior wall engaging members (43) substantially back upon themselves as depicted in Fig. 16.

Following this, the stapling tool (10) may be manipulated to urge the forward end of the tool (10), the forward end of the staple (12), and the everted end of the blood vessel (51) through a hole (52) provided through the wall (53) of a second blood vessel or other appropriate structure as indicated in Fig. 17. The hole (52) provided through the wall (53) should be of an appropriate diameter to accommodate the exterior diameter of the blood vessel (51).

The stapling tool (10) may then be retracted somewhat to cause the plurality of vessel and interior wall engaging members (43) to pierce and become embedded in the interior side of the wall (53) as depicted in Fig. 18. Following this, the trigger unit (17) may be manipulated to cause the sleeve (13) to move forwardly and cause the anvil disposed on its forward end to contact the plurality of exterior wall engaging members (44) and thereby urge them forward into a piercing and engaging orientation as depicted in Fig. 19.

Following this, the mandrel (16) may be gently withdrawn from the stapling tool (10) to allow the fingers (26) of the core unit (14) to retract inwardly somewhat and thereby release their hold on the staple (12). The stapling tool (10) may then be gently withdrawn as a whole, leaving the blood vessel (51) firmly connected to the wall (53) in a stapled anastomosis.

Obviously, numerous modifications and variations of the present invention are possible in light of the above teachings. It is therefore to be understood that within the scope of the appended claims, the invention may be practiced otherwise than as specifically described therein.

## Claims

1. A vascular anastomosis staple (12) for joining one end of a first flexible conduit (51) in a branched connection with a second flexible conduit (53) having an opening (52) formed in the side of the second flexible conduit, the staple comprising:
a substantially rigid annular base member (46) formed about a longitudinal center line and having an open center, an upper end, and a lower end;
a plurality of spaced apart and oppositely facing first (43) and second (44) engaging member means extending initially outwardly from one another in a longitudinal direction which is in the same general plane as said annular base member (46), wherein the first engaging member means (43) are attached on one end thereof to, and extending from the upper end of base member (46) for permitting one end of said first flexible conduit (51) extending through said annular base member (46) to be folded back over and piercingly engaged by the other end of said first engaging member means (43), said first engaging member means (43) extending through the opening (52) in the side of said second flexible conduit (53), and being in engagement with the interior of said second flexible conduit (53) around the opening (52) thereof; and wherein said second engaging member means (44) are attached at one end thereof to, and extending from the lower end of, said base member (46) and being in engagement with the exterior of said second flexible conduit (53) for securing the end of the first flexible conduit (51) in fluid communication with the side of the second flexible conduit (53).

2. The staple (12) of claim 1 wherein said one end of the first (43) and second (44), engaging member means are axially offset relative to one another.

3. The staple (12) of claim 2 wherein said first engaging member means (43) further comprises an intermediate portion disposed between said one end and said other end; and, wherein said other end is disposed at an angle relative to said one end.

4. The staple (12) of claim 3 wherein said intermediate portion is angularly offset from both said one end and said other end.

5. The staple (12) of claim 1 wherein said annular base member (46) has a diameter just slightly larger than the diameter of said first flexible conduit.

6. The staple (12) of claim 1 wherein the other ends of each of said first (43) and second (44) engaging member means are pointed for easily piercing of the flexible conduits.

7. The staple (12) of claim 6 wherein said second engaging member means (44) have a first bend near the other end thereof causing the other end to extend somewhat radially outwardly from the center line on the other end thereof.

8. The staple (12) of claim 7 wherein said second engaging member means (44) have a second bend therein intermediate the first bend and said one end thereof whereby pressure against said second member means at the first bend in a direction along the center line and toward the base member will cause said second engaging member means to fold back over to a position radially outwardly from said base member, for engaging the exterior of said flexible conduit.

9. The staple (12) of claim 1 wherein said first engaging member means (43) are initially straight for piercing the folded back end of the first flexible conduit (51) but then are forcibly folded back over the folded back end of the first flexible conduit (51) whereby the first engaging member means (43) are disposed at least partially radially outwardly from said base member (46) for engaging the interior wall of the second flexible conduit (53) thereby preventing the end of the first flexible conduit (51) from being pulled out of the opening (52) in the side of the second flexible conduit (53).

10. The staple (12) of claim 1 wherein the first (43) and second (44) engaging member means are alternatingly spaced around the periphery of the base member (46) whereby there will be evenly spaced support around the periphery of the staple (12) after it is installed.

## Patentansprüche

1. Klammer (12) für Gefäßanastomose zum Verbinden eines Endes einer ersten flexiblen Leitung (51) in einer verzweigten Verbindung mit einer zweiten flexiblen Leitung (53), die eine Öffnung (52) aufweist, die in der Seite der zweiten flexiblen Leitung ausgebildet ist, wobei die Klammer umfasst:
ein im Wesentlichen starres ringförmiges Sockelelement (46), das um eine Längsmittellinie herum ausgebildet ist und eine offene Mitte, ein oberes Ende und ein unteres Ende aufweist;
eine Vielzahl beabstandeter und entgegengesetzt gerichteter erster (43) und zweiter (44) Eingriffselementeinrichtungen, die sich zunächst voneinander weg nach außen in einer Längsrichtung erstrecken, die in der gleichen allgemeinen Ebene liegt wie das ringförmige Sockelelement (46), wobei die ersten Eingriffselementeinrichtungen (43) an einem Ende derselben an dem Sockelelement (46) angebracht sind und sich von dem oberen Ende desselben ausstrecken, so dass ein Ende der ersten flexiblen Leitung (51), das sich durch das ringförmige Sockelelement (46) hindurch erstreckt, um das andere Ende der ersten Eingriffselementeinrichtungen (43) umgeschlagen werden und durchbohrend damit in Eingriff kommen kann, wobei sich die ersten Eingriffselementeinrichtungen (43) durch die Öffnung (52) in der Seite der zweiten flexiblen Leitung (53) hindurch erstrecken und mit dem Inneren der zweiten flexiblen Leitung (53) um die Öffnung (52) derselben herum in Eingriff kommen, und wobei die zweiten Eingriffselementeinrichtungen (44) an einem Ende derselben an dem Sockelelement (46) angebracht sind und sich von dessen unterem Ende aus erstrecken und mit der Außenseite der zweiten flexiblen Leitung (53) in Eingriff kommen, um das Ende der ersten flexiblen Leitung (51) in Fluidverbindung mit der Seite der zweiten flexiblen Leitung (53) zu halten.

2. Klammer (12) nach Anspruch 1, wobei die einen Enden der ersten (43) und der zweiten (44) Eingriffselementeinrichtungen axial zueinander versetzt sind.

3. Klammer (12) nach Anspruch 2, wobei die erste Eingriffselementeinrichtung (43) des Weiteren einen Zwischenabschnitt umfasst, der zwischen dem einen Ende und dem anderen Ende angeordnet ist, und wobei das andere Ende in einem Winkel in Bezug zu dem einen Ende angeordnet ist.

4. Klammer (12) nach Anspruch 3, wobei der Zwischenabschnitt winklig sowohl zu dem einen Ende als auch zu dem anderen Ende versetzt ist.

5. Klammer (12) nach Anspruch 1, wobei das ringförmige Sockelelement (46) einen Durchmesser hat, der nur geringfügig größer ist als der Durchmesser der ersten flexiblen Leitung.

6. Klammer (12) nach Anspruch 1, wobei die anderen Enden jeder der ersten (43) und der zweiten (44) Eingriffselementeinrichtungen spitz sind, um die flexiblen Leitungen leicht zu durchbohren.

7. Klammer (12) nach Anspruch 6, wobei die zweiten Eingriffselementeinrichtungen (44) eine erste Biegung in der Nähe des anderen Endes derselben haben, die bewirkt, dass sich das andere Ende leicht radial nach außen von der Mittellinie an dem anderen Ende derselben erstreckt.

8. Klammer (12) nach Anspruch 7, wobei die zweiten Eingriffselementeinrichtungen (44) eine zweite Biegung darin zwischen der ersten Biegung und dem einen Ende derselben haben, so dass Druck auf die zweiten Elementeinrichtungen an der ersten Biegung in einer Richtung entlang der Mittellinie und auf das Sockelelement zu bewirkt, dass die zweiten Eingriffselementeinrichtungen über eine Position radial außerhalb des Sockelelementes umgeschlagen werden, um mit der Außenseite der flexiblen Leitung in Eingriff zu kommen.

9. Klammer (12) nach Anspruch 1, wobei die ersten Eingriffselementeinrichtungen (43) zunächst gerade sind, um das umgeschlagene hintere Ende der ersten flexiblen Leitung (51) zu durchbohren, dann jedoch unter Druck über das umgeschlagene hintere Ende der ersten flexiblen Leitung (51) umgeschlagen werden, so dass die ersten Eingriffselementeinrichtungen (43) wenigstens teilweise radial außerhalb des Sockelelementes (46) angeordnet sind, um mit der Innenwand der zweiten flexiblen Leitung (53) in Eingriff zu kommen und so zu verhindern, dass das Ende der ersten flexiblen Leitung (51) aus der Öffnung (52) in der Seite der zweiten flexiblen Leitung (53) herausgezogen wird.

10. Klammer (12) nach Anspruch 1, wobei die ersten (43) und die zweiten (44) Eingriffselementeinrichtungen abwechselnd um den Umfang des Sockelelementes (46) herum beabstandet sind, so dass gleichmäßig beabstandeter Halt um den Umfang der Klammer (12) herum vorhanden ist, nachdem sie installiert worden ist.

## Revendications

1. Agrafe (12) pour anastomose vasculaire destinée à relier une extrémité d'un premier conduit souple (51) dans un raccordement de branchement avec un second conduit souple (53) ayant une ouverture (52) formée dans le côté du second conduit souple, l'agrafe comportant :
un élément de base annulaire sensiblement rigide (46) formé autour d'un axe central longitudinal et ayant un centre ouvert, une extrémité supérieure et une extrémité inférieure ;
plusieurs premiers (43) et seconds (44) moyens à éléments d'engagement espacés et tournés dans des directions opposées, s'étendant initialement vers l'extérieur les uns par rapport aux autres dans une direction longitudinale qui est dans le même plan général que ledit élément de base annulaire (46), les premiers moyens à éléments d'engagement (43) étant reliés par une première de leurs extrémités à, et s'étendant depuis, l'extrémité supérieure dudit élément de base (46), pour permettre à une extrémité dudit premier conduit souple (51) s'étendant à travers ledit élément de base annulaire (46) d'être repliées en arrière sur, et engagée par perçage par, l'autre extrémité desdits premiers moyens à éléments d'engagement (43), lesdits premiers moyens à éléments d'engagement (43) s'étendant à travers l'ouverture (52) dans le côté dudit second conduit souple (53), et étant en prise avec l'intérieur dudit second conduit souple (53) autour de son ouverture (52) ; et dans laquelle lesdits seconds moyens à éléments d'engagement (44) sont attachés par une première de leurs extrémités audit élément de base et s'étendent depuis l'extrémité inférieure dudit élément de base (46) et étant en prise avec l'extérieur dudit second conduit souple (53) pour assujettir l'extrémité du premier conduit souple (51) en communication de fluide avec le côté du second conduit souple (53).

2. Agrafe (12) selon la revendication 1, dans laquelle lesdites premières extrémités des premiers (43) et seconds (44) moyens à éléments d'engagement sont décalées axialement les unes par rapport aux autres.

3. Agrafe (12) selon la revendication 2, dans laquelle lesdits premiers moyens à éléments d'engagement (43) comprennent en outre une partie intermédiaire disposée entre ladite première extrémité et ladite autre extrémité ; et dans laquelle ladite autre extrémité est disposée de façon à former un angle par rapport à ladite première extrémité.

4. Agrafe (12) selon la revendication 3, dans laquelle ladite partie intermédiaire est décalée angulairement à la fois de ladite première extrémité et de ladite autre extrémité.

5. Agrafe (12) selon la revendication 1, dans laquelle ledit élément de base annulaire (46) présente un diamètre qui n'est que légèrement supérieur au diamètre dudit premier conduit souple.

6. Agrafe (12) selon la revendication 1, dans laquelle les autres extrémités de chacun desdits premiers (43) et seconds (44) moyens à éléments d'engagement sont effilées de façon à percer aisément les conduits souples.

7. Agrafe (12) selon la revendication 6, dans laquelle lesdits seconds moyens à éléments d'engagement (44) ont un premier coude proche de leur autre extrémité, amenant l'autre extrémité à s'étendre quelque peu radialement vers l'extérieur depuis l'axe central sur leur autre extrémité.

8. Agrafe (12) selon la revendication 7, dans laquelle lesdits seconds moyens à éléments d'engagement (44) ont un second coude situé entre leur premier coude et leur première extrémité, grâce à quoi une pression contre lesdits seconds moyens à éléments au niveau du premier coude dans une direction suivant l'axe central et vers l'élément de base amène lesdits seconds moyens à éléments d'engagement à être repliés par dessus dans une position située radialement vers l'extérieur dudit élément de base, pour engager l'extérieur dudit conduit souple.

9. Agrafe (12) selon la revendication 1, dans laquelle lesdits premiers moyens à éléments d'engagement (43) sont initialement droits pour percer l'extrémité repliée en arrière du premier conduit souple (51), mais sont ensuite repliés à force en arrière sur l'extrémité repliée en arrière du premier conduit souple (51), grâce à quoi les premiers moyens à éléments d'engagement (43) sont disposés au moins partiellement radialement vers l'extérieur depuis ledit élément de base (46) pour engager la paroi intérieure du second conduit souple (53), empêchant ainsi l'extrémité du premier conduit souple (51) d'être tirée à l'extérieur de l'ouverture (52) dans le côté du second conduit souple (53).

10. Agrafe (12) selon la revendication 1, dans laquelle les premiers (43) et seconds (44) moyens à éléments d'engagement sont espacés de façon alternée le long de la périphérie de l'élément de base (46), de manière à former un support espacé de façon égale le long de la périphérie de l'agrafe (12) après qu'elle a été mise en place.
